# EUROPEAN PATENT SPECIFICATION

(11) **EP 0 570 401 B1**
(45) Date of publication and mention of the grant of the patent: **09.11.1994**
(21) Application number: 92903377.7
(22) Date of filing: 08.01.1992
(51) Int. Cl.: C07D 307/14, C07D 307/22, A61K 31/34

(54) **LIPOXYGENASE-INHIBITING HYDROXAMIC ACID AND N-HYDROXYUREA DERIVATIVES**
Lipoxygenase inhibierende Hydroxamsäure und N-Hydroxyharnstoff-Derivate
DERIVES D'ACIDE HYDROXAMIQUE ET DE N-HYDROXYUREE INHIBITEURS DE LA LIPOXYGENASE

(30) Priority: 07.02.1991 JP 16325/91
(43) Date of publication of application: 24.11.1993
(73) Proprietor: PFIZER INC., New York, N.Y. 10017 (US)
(72) Inventor: STEVENS, Rodney William, Handa-city, Aichi 475 (JP)
(74) Representative: Moore, James William, Dr.
(86) International application number: US9200027
(87) International publication number: WO9213848

(56) References cited:
- EP-A- 0 279 263
- GB-A- 1 044 308
- US-A- 3 393 209
- US-A- 3 917 653
- Helvetica Chimica Acta, vol. 65, no. 5, 28 july 1982, (Basle, CH), J.M.J. TRONCHET et al., pages 1404-1411
- Journal of Carbohydrate Chemistry, vol. 7, no. 1, January 1988, (NewYork, N.Y., US), J.M.J. TRONCHET et al., pages 169-186
- Carbohydrate Research, vol. 113, 1983, (Amsterdam, NL), R.H. FURNEAUX: pages 241-255

## Description

### BACKGROUND OF THE INVENTION

### Field of the Invention

This invention relates to novel hydroxamic acid and N-hydroxyurea derivatives. The compounds of the present invention inhibit the enzyme 5-lipoxygenase, and are useful in the treatment or alleviation of inflammatory diseases, allergy and cardiovascular diseases in mammals. This invention also relates to pharmaceutical compositions comprising such compounds and to the use of such compounds in treating inflammatory diseases, allergy and cardiovascular diseases in mammals. This invention further relates to methods of making such compounds.

Arachidonic acid, a component of phospholipids found in all cell membranes, is metabolized through an array of enzymatic pathways to afford biologically active metabolites, including the leukotrienes, an important class of chemical mediators. In the first step of this metabolism, the enzyme 5-lipoxygenase converts arachidonic acid to 5-hydroperoxyeicosatetraenoic acid (5-HPETE). Leukotrienes have been implicated in the pathophysiology of inflammatory diseases, including rheumatoid arthritis, gout, asthma, ischemic reperfusion injury, psoriasis and inflammatory bowel disease. Any drug that inhibits 5-lipoxygenase is expected to provide significant new therapy for both acute and chronic inflammatory conditions.

### Description of the Related Art

Recently several review articles on lipoxygenase inhibitors have been reported (See H. Masamune et al., Ann. Rep. Med. Chem., **24**, 71-80 (1989) and B.J. Fitzsimmons et al., Leukotrienes and Lipoxygenases, 427-502 (1989).

Compounds of the same general class as the compounds of the present invention are disclosed in EP 279263 A2, EP 196184 A2, JP Kokai 502179/88 and U.S. patent No. 4,822,809.

### SUMMARY OF THE INVENTION

The present invention provides novel hydroxamic acid and N-hydroxyurea derivatives of the formula 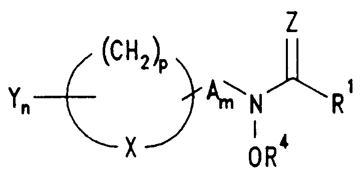
wherein:
R¹ is hydrogen, C1 to C4 alkyl, C2 to C4 alkenyl or NR²R³;
R² and R³ are each independently hydrogen, C1 to C4 alkyl, hydroxy, aryl, or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl, provided that R² and R³ are not both hydroxy;
R⁴ is hydrogen, a pharmaceutically acceptable cation, aroyl or C1 to C12 alkoyl;
A is C1 to C6 alkylene or C2 to C6 alkenylene, provided that A is not alpha to X when m is 0;
X is oxygen of sulfur;
each Y is independently hydrogen, halo, cyano, hydroxy, C1 to C12 alkyl, C1 to C12 alkoxy, C1 to C12 halosubstituted alkyl, C1 to C12 alkylthio, C2 to C12 alkenyl, C2 to C12 alkoxyalkyl, C3 to C8 cyoloalkyl, aryl, aryloxy, C1 to C12 arylalkyl, C2 to C12 arylalkenyl, C1 to C12 arylalkoxy or any of the foregoing aryl-derived moieties substituted with one or more substituents selected from the group consisting of halo, nitro, cyano, C1 to C12 alkyl, C1 to C12 alkoxy, C1 to C12 alkylthio, C1 to C12 alkoyl, C1 to C12 halosubstituted alkyl, aryl, aroyl, aryloxy, arylthio, C1 to C12 arylalkyl, C1 to C12 alkylamino, di C1 to C12 alkylamino, C1 to C12 alkoxycarbonyl, aminocarbonyl, C1 to C12 alkylaminocarbonyl, di C1 to C12 alkylaminocarbonyl and any of the foregoing aroyl or aryl-derived substituents further substituted with one of more substituents selected from the group consisting of halo, nitro, cyano, C1 to C6 alkyl, C1 to C6 alkoxy, C1 to C6 alkylthio and C1 to C6 halosubstituted alkyl;
Z is oxygen or sulfur;
m is 0 or 1;
n is 1 to 5; and
p is 2 to 6.

With regard to the relative orientation of the substituent(s) Y and the linking group A, they may be attached at any available position on the ring.

This invention also concerns pharmaceutical compositions comprising a pharmaceutically acceptable carrier or diluent and a compound of the invention or a pharmaceutically acceptable salt thereof.

### DETAILED DESCRIPTION OF THE INVENTION

### Definitions

As used herein, the following definitions are used.

"Halo" and "halogen" mean radicals derived from the elements fluorine, chlorine, bromine and iodine.

"Alkyl" means straight or branched saturated hydrocarbon radicals, for example, methyl, ethyl, n-propyl and isopropyl.

"Alkenyl" means straight or branched unsaturated hydrocarbon radicals, for example, ethenyl, 1- or 2-propenyl, 2-methyl-1-propenyl and 1- or 2-butenyl.

"Alkylene" means straight or branched saturated hydrocarbon biradicals, for example, -CH₂-, -CH(CH₃)-, -C(CH₃)₂-, -CH₂CH₂-, -CH₂CH(CH₃)-, -CH(C₂H₅)-, -C(CH₃)₂C(CH₃)₂- and -CH₂CH₂CH₂-.

"Alkenylene" means straight or branched unsaturated hydrocarbon biradicals, for example, -CH=CH-, -CH=CHCH₂, -CH=CHCH(CH₃)-, -C(CH₃)=CHCH₂- and -CH₂CH=CHCH₂-.

"Alkoxy" means -OR⁵ wherein R⁵ is an alkyl radical, for example, methoxy, ethoxy, propoxy, isopropoxy and butoxy.

"Alkoxyalkyl" means -R⁶OR⁷ wherein R⁶ and R⁷ are independently alkyl radicals, for example, methoxymethyl, methoxyethyl, ethoxymethyl and ethoxyethyl.

"Alkylthio" means -SR⁸ wherein R⁸ is an alkyl radical, for example, methylthio, ethylthio, propylthio and butylthio.

"Alkylamino" means -NHR⁹ wherein R⁹ is an alkyl radical, for example, methylamino, ethylamino, propylamino and butylamino.

"Dialkyamino" means -NR¹⁰R¹¹ wherein R¹⁰ and R¹¹ are alkyl radicals, for example, dimethylamino, methylethylamino and diethylamino.

"Alkoyl" means -COR¹² wherein R¹² is an alkyl radical, for example, formyl, acetyl, propionyl, butyryl and isobutyryl.

"Aryl" means aromatic radicals, for example, phenyl, naphthyl, pyridyl, quinolyl, thienyl, furyl and phenoxyphenyl.

"Aryloxy" means -OR₁₃ wherein R₁₃ is an aryl radical, for example, phenoxy, 1-naphthoxy and 2-naphthoxy.

"Aroyl" means -COR¹⁴ wherein R¹⁴ is an aryl radical, for example, benzoyl and naphthoyl.

"Arylalkyl" means an aryl group appended to an alkyl radical, for example, benzyl, phenylethyl, 2-phenylethyl, phenylpropyl and 2-pyridylmethyl.

"Arylalkenyl" means an aryl group appended to an alkenyl radical, for example, phenylethenyl and 2-pyridylethenyl.

"Arylalkoxy" means an aryl group appended to an alkoxy radical, for example, benzyloxy and naphthylmethoxy.

"Arylthio" means -SR₁₅ wherein R₁₅ is an aryl radical, for example, phenylthio and naphthylthio.

"Alkoxycarbonyl" means -C(=0)R¹⁶ wherein R¹⁶ is an alkoxy radical, for example, methoxycarbonyl, ethoxycarbonyl and propoxycarbonyl.

"Alkylaminocarbonyl" means -C(=O)NHR¹⁷ wherein R¹⁷ is an alkyl radical, for example, methylaminocarbonyl, ethylaminocarbonyl and propylaminocarbonyl.

"Dialkylaminocarbonyl" means -C(=O)NR¹⁸R¹⁹ wherein R¹⁸ and R¹⁹ are independently alkyl radicals, for example, dimethylaminocarbonyl, diethylaminocarbonyl and methylethylaminocarbonyl.

"Alkylsulfonyl" means -SO₂R²⁰ wherein R²⁰ is an alkyl radical, for example, methanesulfonyl (mesyl) and ethanesulfonyl.

"Halosubstituted alkyl" means an alkyl radical as described above substituted with one or more halogens, for example, chloromethyl, trifluoromethyl and 2,2,2-trichloroethyl.

"Hydroxysubstituted alkyl" means an alkyl radical as described above substituted with one or more hydroxy radicals, for example, hydroxymethyl, dihydroxyethyl and trihydroxypropyl.

"Cycloalkyl" means a carbocyclic radical, for example, cyclopropyl, cyclobutyl, cyclopentyl and cyclohexyl.

"Pharmaceutically acceptable cation" means non-toxic cations based on alkali and alkaline earth metals, for example, sodium, lithium, potassium, calcium and magnesium, as well as non-toxic ammonium, quaternary ammonium and amine cations, for example, ammonium, tetramethylammonium, methylamine, dimethylamine, trimethylamine, ethylamine, diethylamine and triethylamine.

Some of the compounds of the above formula may form acid salts. The pharmaceutically acceptable acid salts are those formed from acids which form non-toxic acid salts, for example, hydrochloride, hydrobromide, sulfate, bisulfate, phosphate, acid phosphate, acetate, citrate, fumarate, gluconate, lactate, maleate, succinate, tartrate, methanesulfonate, benzenesulfonate, toluenesulfonate and formate salts.

### Methods of Preparation

The compounds of the invention may be prepared by a number of synthetic methods. As used in the following reaction schemes, Q is 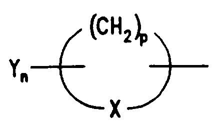
and X, Y, m, n and p are as defined above. Although, in Schemes 1 and 2 below, R¹ is methyl and NH₂, respectively, and Z is oxygen, compounds wherein R¹ and Z are otherwise, as defined above, may be prepared in a similar manner.

In one embodiment, compounds of formula III are prepared according to the reaction steps outlined in Scheme 1. 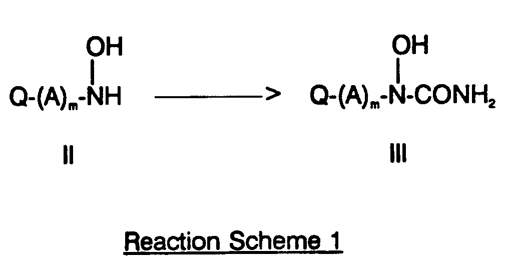
In this step the hydroxylamine (II) is treated with trimethylsilyl isocyanate in a reaction-inert solvent, usually at ambient to reflux temperature. Suitable solvents which do not react with reactants and/or products include, for example, tetrahydrofuran, dioxane, methylene chloride and benzene. An alternative procedure employs treatment of the hydroxylamine (II) with gaseous hydrogen chloride in a reaction-inert solvent such as benzene or toluene followed by treatment with phosgene. Reaction temperatures are usually in the range of ambient temperature to boiling point of solvent. The intermediate carbamoyl chloride is not isolated but is subjected to (i.e. *in situ*) reaction with aqueous ammonia. Furthermore, the hydroxylamine (II) may be treated with aqueous hydrochloric acid in the presence of an alkali metal cyanate such as potassium cyanate in a suitable solvent such as tetrahydrofuran. The product (III) thus obtained is isolated by standard methods and purification can be achieved by conventional means, such as recrystallization and chromatography.

In another embodiment, compounds of the formula V, below, are prepared as illustrated in Scheme 2. 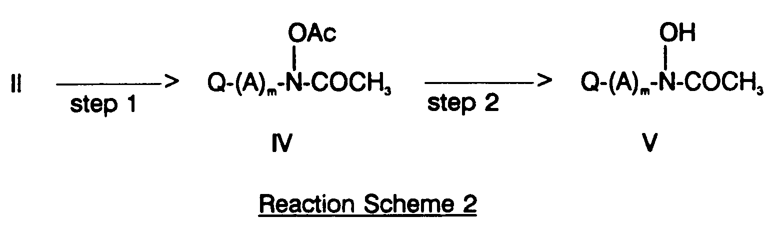
In step 1, the diacetyl compound (IV) is prepared by standard methods known in the art. For example, the hydroxylamine (II) is reacted with acetyl chloride or acetic anhydride in a reaction-inert solvent in the presence of a suitable base. Preferred bases are sodium hydride, triethylamine and pyridine, with the latter two being particularly preferred. Suitable reaction-inert solvents include methylene chloride, chloroform, tetrahydrofuran, benzene and toluene. The reaction is usually carried out in the temperature range of about 0°C through to ambient temperature, with reaction times from 30 minutes to a few hours being typical. The product can be isolated and purified by conventional procedures, such as recrystallization or chromatography.

Step 2 involves selective hydrolysis of the diacetyl (IV) with an appropriate base. Typical bases include ammonium hydroxide, sodium hydroxide, potassium hydroxide and lithium hydroxide, preferably in methanol, ethanol, isopropyl alcohol or water, although binary solvent systems such as alcohol-water, tetrahydrofuran-water and the like may also be employed. Reaction temperatures are usually in the range of about -10°C through to ambient temperature, with the reaction usually complete within a few minutes to several hours. The product (V) is isolated by standard methods and purification can be achieved by conventional means, such as recrystallization and chromatography.

The hydroxylamine (II) can be prepared by treating the corresponding alcohol with N,O-bis(*tert*-butyloxycarbonyl)hydroxylamine under Mitsunobu-type reaction conditions followed by acid-catalyzed hydrolysis, for example, employing trifluoroacetic acid, of the N,O-protected intermediate product (see JP 1045344). It is also noteworthy that the N,O-diacetyl compound (IV) can be prepared employing N,O-diacetylhydroxylamine in place of N,O-bis(*tert*-butyloxycarbonyl)hydroxylamine, thus providing a convenient route to the product of formula V.

Alternatively, the aforementioned hydroxylamine (II) may also be prepared from a suitable halide compound by reaction with an O-protected hydroxylamine and subsequent deprotection. See W.P. Jackson et al., J. Med. Chem., **31**, 499 (1988). Preferred O-protected hydroxylamines include O-tetrahydropyranyl-, O-trimethylsilyl- and O-benzylhydroxylamine.

The hydroxylamine (II) can also be prepared by standard synthetic procedures from readily available carbonyl compounds, for example, ketones, aldehydes and halogen compounds. For example, a suitable carbonyl compound is converted to its oxime and is then reduced to the requisite hydroxylamine (II) with a suitable reducing agent. See, for example, R.F. Borch et al., J. Am. Chem. Soc., **93**, 2897 (1971). Preferred reducing agents include sodium cyanoborohydride and borane complexes such as boron-pyridine, boron-triethylamine and boron-dimethylsulfde; triethylsilane in trifluoroacetic acid may also be employed.

The hydroxylamine (II) thus obtained by the abovementioned representative procedures is isolated by standard methods and purification can be achieved by conventional means, such as recrystallization and chromatography.

The pharmaceutically acceptable salts of the novel compounds of the present invention are readily prepared by contacting said compounds with a stoichiometric amount of, in the case of a non-toxic cation, an appropriate metal hydroxide, alkoxide or amine in either aqueous solution or a suitable organic solvent. In the case of non-toxic acid salt, an appropriate mineral or organic acid in either aqueous solution or a suitable organic solvent can be used. The salt may then be obtained by precipitation or by evaporation of the solvent.

### Biological Activity

The compounds of this invention inhibit lipoxygenase. This inhibition has been demonstrated by an assay using rat peritoneal cavity-resident cells which determines the effect of such compounds on the metabolism of arachidonic acid.

The compounds of the examples were tested according to the methods described in "Synthesis of leukotrienes by peritoneal macrophages", Jap. J. Inflammation, **7**, 145-150 (1987), and were shown to be lipoxygenase inhibitors. In this test some preferred compounds exhibit low IC₅₀ values, in the range of about 0.01 to about 30 µM, for inhibition of lipoxygenase.

The ability of the compounds of the present invention to inhibit lipoxygenase makes them useful for controlling the symptoms induced by the endogenous metabolites arising from arachidonic acid in a mammalian subject. The compounds are therefore valuable in the prevention and treatment of such disease states in which the accumulation of arachidonic acid metabolites is the causative factor, e.g., allergic bronchial asthma, skin disorders, rheumatoid arthritis, osteoarthritis and thrombosis.

The compounds of the formula and their pharmaceutically acceptable salts are of particular use in the prevention and treatment of inflammatory diseases, allergy and cardiovascular diseases in a human subject as well as in the inhibition of lipoxygenase.

### Methods of Administration

For treatment of the various conditions described above, the compounds of the invention and their pharmaceutically acceptable salts can be administered to a human subject either alone or, preferably, in combination with pharmaceutically acceptable carriers or diluents in a pharmaceutical composition, according to standard pharmaceutical practice. A compound can be administered via a variety of conventional routes of administration including orally, parenterally and by inhalation. When the compounds are administered orally, the dose range will generally be from about 0.1 to about 20 mg/kg/day, based on the body weight of the subject to be treated, preferably from about 0.1 to about 1.0 mg/kg/day in single or divided doses. If parenteral administration is desired, then an effective dose will generally be from about 0.1 to about 1.0 mg/kg/day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically acceptable salts can be administered, for example, in the form of tablets, powders, lozenges, syrups or capsules, or as an aqueous solution or suspension. In the case of tablets for oral use, carriers which are commonly used include lactose and corn starch. Lubricating agents, such as magnesium stearate, are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added.

For intramuscular, intraperitoneal, subcutaneous and intravenous use, a sterile solution of the active ingredient is usually prepared, and the pH of the solutions should be suitably adjusted and buffered. For intravenous use, the total concentration of solute should be controlled to make the preparation isotonic.

### Examples

The present invention is illustrated by the following examples. However, it should be understood that the invention is not limited to specific details of these examples. Proton nuclear magnetic resonance (NMR) spectra were measured at 270 MHz unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad. The *cis/trans* isomers referred to in the examples are with regard to the relative orientation of the A and Y substituents on the central heterocyclic ring.

### Example 1 N-hydroxy-N-[5-phenyltetrahydrofuran-2-yl)methylurea

A is 2-methylene, R¹ is NH₂, R⁴ is hydrogen, X is oxygen, Y is 5-phenyl, Z is oxygen, m is 1, n is 1 and p is 4 (trans/cis isomers present in 35:65 ratio).

### Step 1, 5-phenyltetrahydrofurfuryl alcohol

A dichloromethane solution (25 ml) of *m*-chloroperoxybenzoic acid (4.85 g, 28 mmol) was added over 10 minutes to a solution of 1-phenyl-4-penten-1-ol (3.8 g, 23 mmol, prepared according to the procedure of L. Overman et al, J. Am. Chem. Soc., **112**, 3945 (1990)) in 10 ml dichloromethane at under 20°C. After stirring for greater than 10 hours at ambient temperature, camphor sulfonic acid (50 mg) was added and stirring was continued for 3 hours. The precipitated *m*-chlorobenzoic acid was removed by filtration and the filtrate washed with saturated sodium bicarbonate solution (2 x 10 ml), dried over MgSO₄ and concentrated. The resultant oil was purified by flash chromatography (silica gel, hexane/diethyl ether, 2:1), affording 3.09 g (74% yield) of the title compound as a colorless oil.
¹H NMR (CDCl₃) δ 7.4-7.2 (m, 5H), 5.05-4.85 (m, 1H), 4.4-4.3 (m) and 4.25-4.15 (m, 1H), 3.83-3.52 (m, 2H), 2.4-2.25 (m, 2H), 2.1-1.98 (m, 1H), 1.9-1.75 (m, 2H).

### Step 2, N,O-bis(tert-butoxycarbonyl)-(5-phenyltetrahydrofuran-2-yl)methylhydroxylamine

To a stirred solution of the product of Step 1, above, (2.89 g, 16 mmol), N,O-bis-(*tert*-butoxycarbonyl)hydroxylamine (3.78 g, 16 mmol) and triphenylphosphine (5.53 g, 21 mmol) in tetrahydrofuran (40 ml) at 0°C, was added dropwise a solution of diethyl azodicarboxylate (3.67 g, 21 mmol) in tetrahydrofuran (10 ml). The mixture was then warmed to room temperature, stirred for 1 hour and the volatiles were removed under reduced pressure. The residue was purified by flash chromatography (silica gel, hexane/ethyl acetate, 13:1), affording 4.2 g (67% yield) of the title compound as a pale yellow oil.
IR (film) v 2980, 1780, 1720 cm⁻¹.
¹H NMR (CDCl₃) δ 7.37-7.21 (m, 5H), 5.02 (t, J=7 Hz) and 4.89 (t, J=7 Hz, 1H), 4.5-4.43 (m) and 4.35-4.25 (m, 1H), 3.84-3.7 (m, 2H), 2.45-2.1 (m, 2H), 1.92-1.76 (m, 2H), 1.49 (s, 9H), 1.48 (s, 9H).

### Step 3, N-hydroxy-N-(5-phenyltetrahydrofuran-2-yl)methylurea

To a vigorously stirred solution of the product of Step 2, above, (4.02 g, 10 mmol) in dichloromethane (25 ml) was added dropwise trifluoroacetic acid (8 ml). After the reaction was complete, as determined by thin layer chromatography, the reaction mixture was poured carefully into a solution of sodium carbonate (50 ml). The organic layer was separated, the aqueous layer was extracted with dichloromethane (2 x 15 ml) and the combined organic layers were dried over MgSO₄ and concentrated. The resultant oil was dissolved in tetrahydrofuran (25 ml) and trimethylsilylisocyanate (2.43 ml, 20 mmol) was added. After stirring for 1 hour, volatiles were removed under reduced pressure and the resultant residue reecrystallized from dichloromethane, affording 327 mg (14% yield) of the title compound as a white solid, m.p. 115.4-116.3°C.
IR (KBr) v 3450, 3320, 2880, 1630 cm⁻¹.
¹H NMR (DMSO-d₆) δ 9.38 (s, 1H), 7.37-7.24 (m, 5H), 6.28 (s, 2H), 4.92 (t, J=6 Hz) and 4.84 (t, J=6 Hz, 1H), 4.4-4.34 (m) and 4.25-4.15 (m, 1H), 3.64-3.5 (m, 1H), 3.4-3.3 (m, 1H), 2.35-2.2 (m, 1H), 2.1-1.98 (m, 1H), 1.8-1.58 (m, 2H).

### Example 2 N-hydroxy-N-[5-(3-phenoxy)-phenyltetrahydrofuran-2-yl]methylurea

A is 2-methylene, R¹ is NH₂, R⁴ is hydrogen, X is oxygen, Y is 5-(3-phenoxyphenyl), Z is oxygen, m is 1, n is 1 and p is 4 (trans/cis isomers present in 50:50 ratio).

The title compound, m.p. 95.5-96.2°C, was prepared from 1-(3-phenoxyphenyl)-4-penten-1-ol according to the procedure of Example 1, above.
IR (KBr) v 3310, 2890, 1630, 1485 cm⁻¹.
¹H NMR (DMSO-d₆) δ 9.37 (s) and 9.35 (s, 1H), 7.42-7.3 (m, 3H), 7.39 (t, J=7 Hz, 3H), 7.32 (dd, J=9, 1.8 Hz, 1H), 7.14 (t, J=7 Hz, 2H), 7.07-6.96 (m, 1H), 6.88-6.84 (m, 1H), 6.25 (s, 2H), 4.92 (t, J=7 Hz) and 4.82 (t, J=7 Hz, 1H), 4.4-4.3 (m) and 4.2-4.1 (m, 1H), 3.64-3.5 (m, 1H), 3.4-3.3 (m, 1H), 2.35-2.2 (m, 1H), 2.1-1.95 (m, 1H), 1.8-1.58 (m, 2H).

### Example 3 N-hydroxy-N-[5-[2-phenylethyl)tetrahydrofuran-2-yl]methylurea

A is 2-methylene, R¹ is NH₂, R⁴ is hydrogen, X is oxygen, Y is 5-(2-phenylethyl), Z is oxygen, m is 1, n is 1 and p is 4 (trans/cis isomers present in 50:50 ratio).

The title compound, m.p. 107.5-108.5°C, was prepared from 1-phenyl-6-hepten-3-ol according to the procedure of Example 1, above.
IR (KBr) v 3300, 2875, 1635 cm⁻¹.
¹H NMR (DMSO-d₆) δ 9.31 (s, 1H), 7.3-7.13 (m, 5H), 6.24 (s, 2H), 4.2-4.1 (m) and 4.05-3.95 (m, 1H), 3.85-3.8 (m) and 3.8-3.65 (m, 1H), 3.54-3.44 (m, 1H), 3.25-3.15 (m, 1H), 2.7-2.55 (m, 1H), 2.47 (s, 1H), 2.05-1.4 (m, 6H).

### Example 4 trans-N-hydroxy-N-(5-phenyltetrahydrofuran-2-yl)methylurea

A is 2-methylene, R¹ is NH₂, R⁴ is hydrogen, X is oxygen, Y is 5-phenyl, Z is oxygen, m is 1, n is 1 and p is 4.

The title compound, m.p. 99.5-100.1°C, was prepared from *trans*-5-phenyltetrahydrofurfuryl alcohol according to the procedure of Example 1, above. *Trans*-5-phenyltetrahydrofurfuryl alcohol was prepared from 1-phenyl-4-penten-1-ol according to the procedure of S. Onaki et al, Chem. Lett., **67** (1990).
IR (KBr) v 3300, 2750, 1640 cm⁻¹.
¹H NMR (DMSO-d₆) δ 9.37 (s, 1H), 7.33-7.23 (m, 5H), 6.27 (s, 2H), 4.92 (t, J=7 Hz, 1H), 4.4-4.3 (m, 1H), 3.68 (dd, J=14, 6.5 Hz, 1H), 3.3 (dd, J=14, 6.5 Hz, 1H), 2.36-2.25 (m, 1H), 2.1-1.98 (m, 1H), 1.8-1.6 (m, 2H).

### Example 5 cis-N-hydroxy-N-(5-phenyltetrahydrofuran-2-yl)methylurea

A is 2-methylene, R¹ is NH₂, R⁴ is hydrogen, X is oxygen, Y is 5-phenyl, Z is oxygen, m is 1, n is 1 and p is 4.

The title compound, m.p. 133.2-134.3°C, was prepared according to the procedure of Example 4, above.
IR (KBr) v 3450, 3300, 2875, 1630, 1500, 1200, 760, 700 cm⁻¹.
¹H NMR (DMSO-d₆) δ 9.39 (s, 1H), 7.36-7.24 (m, 5H), 6.27 (s, 2H), 4.82 (t, J=7 Hz, 1H), 4.21 (quint., J=7 Hz, 1H), 3.60 (dd, J=14, 6 Hz, 1H), 3.41 (dd, J=14, 6 Hz, 1H), 2.3-2.2 (m, 1H), 2.1-1.98 (m, 1H), 1.8-1.6 (m, 2H).

### Example 6 trans-N-hydroxy-N-[5-(3-phenoxy)phenyltetrahydrofuran-2-yl]methylurea

A is 2-methylene, R¹ is NH₂, R⁴ is hydrogen, X is oxygen, Y is 5-(3-phenoxyphenyl), Z is oxygen, m is 1, n is 1 and p is 4.

The title compound, a viscous oil, was prepared according to the procedure of Example 4, above.
¹H NMR (DMSO-d₆) δ 9.36 (s, 1H), 7.36 (m, 3H), 7.16 (d, J=7 Hz, 1H), 7.11 (t, J=7 Hz, 1H), 7.01 (d, J=8 Hz, 2H), 6.98 (d, J=2 Hz, 1H), 6.86 (dd, J=8, 2 Hz, 1H), 6.23 (s, 2H), 4.91 (t, J=7 Hz, 1H), 4.34 (quint., J= 7 Hz, 1H), 3.60 (dd, J=14, 6 Hz, 1H), 3.27 (dd, J=14, 6 Hz, 1H), 2.37-2.26 (m, 1H), 2.1-1.95 (m, 1H), 1.75-1.6 (m, 2H).

### Example 7 trans-N-hydroxy-N-[5-(4-fluorophenyl)tetrahydrofuran-2-yl]methylurea

A is 2-methylene, R¹ is NH₂, R⁴ is hydrogen, X is oxygen, Y is 5-(4-fluorophenyl), Z is oxygen, m is 1, n is 1 and p is 4.

The title compound, m.p. 137.6-138.1°C, was prepared according to the procedure of Example 4, above.
IR (KBr) v 3450, 3300, 2875, 1640, 1605, 1510, 1480, 1220, 695 cm⁻¹.
¹H NMR (DMSO-d₆) δ 9.36 (s, 1H), 7.35 (dd, J=9, 6 Hz, 2H), 7.14, (t, J=9 Hz, 2H), 6.26 (s, 2H), 4.92 (t, J=7 Hz, 1H), 3.65 (quint., J=7 Hz, 1H), 3.61 (dd, J=14, 6 Hz, 1H), 3.28 (dd, J=14, 6 Hz, 1H), 2.35-2.25 (m, 1H), 2.1-2.0 (m, 1H), 1.8-1.6 (m, 2H).

### Example 8 trans-N-hydroxy-N-[5-(4-chlorophenyl)tetrahydrofuran-2-yl]methylurea

A is 2-methylene, R¹ is NH₂, R⁴ is hydrogen, X is oxygen, Y is 5-(4-chlorophenyl), Z is oxygen, m is 1, n is 1 and p is 4.

The title compound, m.p. 160.9-161.9°C, was prepared according to the procedure of Example 4, above.
IR (KBr) v 3300, 2875, 1630, cm⁻¹.
¹H NMR (DMSO-d₆) δ 9.36 (s, 1H), 7.4-7.31 (m, 4H), 6.27 (s, 2H), 4.93 (t, J=6 Hz, 1H), 4.38 (quint., J=7 Hz, 1H), 3.61 (dd, J=14, 6 Hz, 1H), 3.29 (dd, J=14, 6 Hz, 1H), 2.4-2.35 (m, 1H), 2.1-1.95 (m, 1H), 1.8-1.6 (m, 2H).

### Example 9 N-hydroxy-N-(4-phenyltetrahydrofuran-2-yl)methylurea

A is 2-methylene, R¹ is NH₂, R⁴ is hydrogen, X is oxygen, Y is 4-phenyl, Z is oxygen, m is 1, n is 1 and p is 4.

The title compound, m.p. 134.8-135.6°C, was prepared according to the procedure of Example 1, above.
IR (KBr) v 3500, 3300, 2875, 1650, 1590, 1560, 1495, 765, 705 cm⁻¹.
¹H NMR (DMSO-d₆) δ 9.36 (s, 1H), 7.34-7.21 (m, 5H), 6.27 (s, 2H), 4.35-4.18 (m, 1H), 4.12 and 4.03 (t, J=7 Hz, 1H), 3.75-3.3 (m, 4H), 2.5-2.36 and 1.75-1.62 (m, 1H), 2.15-2.0 (m, 1H).

### Example 10 trans-N-hydroxy-N-(5-[3-(4-methylphenoxy)phenyl]tetrahydrofuran-2-yl)methylurea

A is 2-methylene, R¹ is NH₂, R⁴ is hydrogen, X is oxygen, Y is 5-[3-(4-methylphenoxy)phenyl], Z is oxygen, m is 1, n is 1 and p is 4.

The title compound, a viscous oil, was prepared according to the procedure of Example 4, above.
¹H NMR (DMSO-d₆) δ 9.37 (s, 1H), 7.31 (t, J=8 Hz, 1H), 7.18 (d, J=8 Hz, 2H), 7.04 (d, J=8 Hz, 1H), 6.92 (s, 1H), 6.90 (d, J=8 Hz, 2H), 6.81 (dd, J=8, 2 Hz, 1H), 6.27 (s, 2H), 4.90 (t, J=7 Hz, 1H), 4.34 (quint., J= 7 Hz, 1H), 3.60 (dd, J=14, 6 Hz, 1H), 3.28 (dd, J=14, 6 Hz, 1H), 2.35-2.2 (m, 1H), 2.29 (s, 3H), 2.1-1.97 (m, 1H), 1.75-1.6 (m, 2H).

### Example 11 N-hydroxy-N-[5-(4-phenoxyphenyl)tetrahydrofuran-2-yl]methylurea

A is 2-methylene, R¹ is NH₂, R⁴ is hydrogen, X is oxygen, Y is 5-(4-phenoxyphenyl), Z is oxygen, m is 1, n is 1 and p is 4.

The title compound, m.p. 143.4-144.4°C, was prepared according to the procedure of Example 2, above.
IR (KBr) v 3300, 2875, 1635, 1595, 1490, 1240 cm⁻¹.
¹H NMR (DMSO-d₆) δ 9.34 (s, 1H), 7.39 (d, J=7 Hz, 2H), 7.33 (d, J=7 Hz, 2H), 7.12 (t, J=7 Hz, 1H), 6.97 (t, J=7 Hz, 4H), 6.27 (s, 2H), 4.94 and 4.84 (t, J=6 Hz, 1H), 4.37 and 4.20 (quint., J=7 Hz, 1H), 3.65-3.55 (m, 1H), 3.45-3.35 (m, 1H), 2.35-2.20 (m, 1H), 2.1-2.0 (m, 1H), 1.8-1.63 (m, 2H).

### Example 12 N-hydroxy-N-[4-(2-phenyl)tetrahydrofuranyl]urea

R¹ is NH₂, R⁴ is hydrogen, X is oxygen, Y is 2-phenyl, Z is oxygen, m is 0, n is 1 and p is 4.

### Step 1, 4-hydroxylamino-2-phenyltetrahydrofuran

To a solution of 2-phenyltetrahydrofuran-4-one (1.3 g, 8 mmol, prepared according to the method of G.W.L. Ellis et al., Can. J. Chem., **63** (12), 3510-3515 (1985)) in pyridine (15 ml) was added hydroxylamine hydrochloride (0.83 g, 12 mmol). The reaction mixture was stirred at room temperature overnight and was then diluted with water and extracted with dichloromethane (2 x 30 ml). The extract was washed with water (30 ml) and brine (30 ml) and was dried over MgSO₄. After removal of solvent, the crude oxime was obtained as a pale yellow solid (1.4 g). To a solution of the crude oxime in acetic acid (15 ml) was added NaBH₃CN (1.9 g, 30 mmol) portionwise in solid form. After stirring for 30 minutes, the reaction mixture was poured carefully into ice cold saturated aqueous NaHCO₃ (50 ml) and was extracted with dichloromethane (2 x 50 ml). The combined extracts were washed with water (50 ml) and brine (50 ml), then dried over MgSO₄ and the solvent was removed under reduced pressure. Chromatography on silica gel of the resultant residue (ethyl acetate/n-hexane, 1:1) afforded the title compound (0.91 g, 65% yield).
¹H NMR (CDCl₃) δ 7.32 (m, 5H), 5.05 (dd, J=6.60, 9.16 Hz, 0.4H), 4.82 (t, J=7.70 Hz, 0.6H), 4.20 (m, 1H), 3.89 (m, 2H), 3.35 (br s, 2H), 2.57 (m, 0.6H), 2.31 (m, 0.4H), 1.97 (m, 0.4H), 1.68 (m, 0.6H).

### Step 2, N-hydroxy-N-[4-[2-phenyl)tetrahydrofuranyl]urea

To a solution of the product of Step 1, above (0.91 g, 5 mmol), in tetrahydrofuran (10 ml) was added trimethylsilylisocyanate (0.67 g, 5.5 mmol) and the reaction mixture was stirred at room temperature for 1.5 hours. Methanol (10 ml) was added and after stirring for 10 minutes, solvent was removed under reduced pressure to afford white solids. Recrystallization from ethyl acetate/n-hexane afforded the title compound as a colorless solid (0.45 g, 54% yield), m.p. 132.1-133.6°C.
IR (KBr) v 3450, 3200, 2900, 1610, 1570, 1490, 1070 cm⁻¹.
¹H NMR (DMSO-d₆) δ 9.28 (s, 0.5H), 9.25 (s, 0.5H), 7.31 (m, 5H), 6.43 (s, 1H), 6.39 (s, 1H), 4.91 (m, 1.5H), 4.73 (m, 0.5H), 4.13 (m, 0.5H), 3.88 (m, 1H), 3.76 (m, 0.5H), 2.34 (m, 1H), 1.84 (m, 1H).

## Claims (Claims for the following Contracting State(s): AT, BE, CH, DE, DK, FR, GB, IT, LI, LU, NL, SE)

1. A compound of the formula 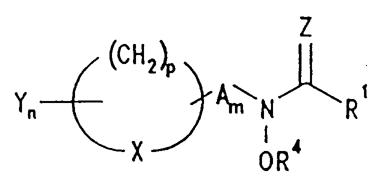 wherein:
R¹ is hydrogen, C1 to C4 alkyl, C2 to C4 alkenyl or NR²R³;
R² and R³ are each independently hydrogen, C1 to C4 alkyl, hydroxy, aryl, or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl, provided that R² and R³ are not both hydroxy;
R⁴ is hydrogen, a pharmaceutically acceptable cation, aroyl or C1 to C12 alkoyl;
A is C1 to C6 alkylene or C2 to C6 alkenylene, provided that A is not alpha to X when m is 0;
X is oxygen or sulfur;
each Y is independently hydrogen, halo, cyano, hydroxy, C1 to C12 alkyl, C1 to C12 alkoxy, C1 to C12 halosubstituted alkyl, C1 to C12 alkylthio, C2 to C12 alkenyl, C2 to C12 alkoxyalkyl, C3 to C8 cycloalkyl, aryl, aryloxy, C1 to C12 arylalkyl, C2 to C12 arylalkenyl, C1 to C12 arylalkoxy or any of the foregoing aryl-derived moieties substituted with one or more substituents selected from the group consisting of halo, nitro, cyano, C1 to C12 alkyl, C1 to C12 alkoxy, C1 to C12 alkylthio, C1 to C12 alkoyl, C1 to C12 halosubstituted alkyl, aryl, aroyl, aryloxy, arylthio, C1 to C12 arylalkyl, C1 to C12 alkylamino, di C1 to C12 alkylamino, C1 to C12 alkoxycarbonyl, aminocarbonyl, C1 to C12 alkylaminocarbonyl, di C1 to C12 alkylaminocarbonyl and any of the foregoing aroyl or aryl-derived substituents further substituted with one or more substituents selected from the group consisting of halo, nitro, cyano, C1 to C6 alkyl, C1 to C6 alkoxy, C1 to C6 alkylthio and C1 to C6 halosubstituted alkyl;
Z is oxygen or sulfur;
m is 0 or 1;
n is 1 to 5; and
p is 2 to 6.

2. A compound according to Claim 1 wherein R⁴ is hydrogen.

3. A compound according to Claim 1 or 2 wherein:
p is 4; and
X is oxygen.

4. A compound according to one or Claims 1 to 3 wherein:
R¹ is NH₂; and
Z is oxygen.

5. A compound according to one of Claims 1 to 4 wherein:
Y is aryl, substituted aryl or arylalkyl; and
n is 1.

6. A compound according to Claim 5 selected from the group consisting of:
N-hydroxy-N-(5-phenyltetrahydrofuran-2-yl)methylurea;
N-hydroxy-N-[5-(3-phenoxy)phenyltetrahydrofuran-2-yl]methylurea;
N-hydroxy-N-[5-(2-phenylethyl)tetrahydrofuran-2-yl]methylurea;
N-hydroxy-N-(4-phenyltetrahydrofuran-2-yl)methylurea;
N-hydroxy-N-[5-(4-phenoxyphenyl)tetrahydrofuran-2-yl]methylurea;
and
N-hydroxy-N-[4-(2-phenyl)tetrahydrofuranyl]urea.

7. A compound according to Claim 6 selected from the group consisting of:
*trans*-N-hydroxy-N-(5-phenyltetrahydrofuran-2-yl)methylurea;
*cis*-N-hydroxy-N-(5-phenyltetrahydrofuran-2-yl)methylurea; and
*trans*-N-hydroxy-N-[5-(3-phenoxy)phenyltetrahydrofuran-2-yl]methylurea.

8. A compound according to Claim 5 selected from the group consisting of:
*trans*-N-hydroxy-N-[5-(4-fluorophenyl)tetrahydrofuran-2-yl]methylurea;
*trans*-N-hydroxy-N-[5-(4-chlorophenyl)tetrahydrofuran-2-yl]methylurea;
and
*trans*-N-hydroxy-N-(5-[3-(4-methylphenoxy)phenyl]tetrahydrofuran-2-yl)methylurea.

9. A pharmaceutical composition for the treatment of allergic or inflammatory conditions in a mammal comprising a therapeutically effective amount of a compound according to one of Claims 1 to 8, or a pharmaceutically acceptable salt thereof, and a pharmaceutically acceptable carrier.

10. A pharmaceutical composition for the treatment of cardiovascular diseases in a mammal comprising a therapeutically effective amount of a compound according to one of Claims 1 to 8, or a pharmaceutically acceptable salt thereof, and a pharmaceutically acceptable carrier.

11. A compound according to any one of claims 1 to 8, or a pharmaceutically acceptable salt thereof, or a pharmaceutical composition containing either entity, for use as a medicament.

12. The use of a compound according to any one of claims 1 to 8, or a pharmaceutically acceptable salt thereof, or a pharmaceutical composition containing either entity, for the manufacture of a medicament for inhibiting lipoxygenase.

13. The use of a compound according to any one of claims 1 to 8, or a pharmaceutically acceptable salt thereof, or a pharmaceutical composition containing either entity, for the manufacture of a medicament for the treatment of allergy or inflammatory conditions.

14. The use of a compound according to any one of claims 1 to 8, or a pharmaceutically acceptable salt thereof, or a pharmaceutical composition containing either entity, for the manufacture of a medicament for the treatment of cardiovascular diseases.

## Claims (Claims for the following Contracting State(s): ES, GR)

1. A process for preparing a compound of the formula 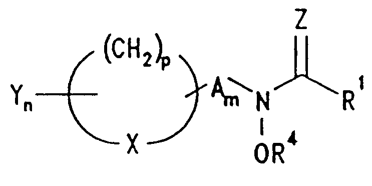 wherein:
R¹ is hydrogen, C1 to C4 alkyl, C2 to C4 alkenyl or NR²R³;
R² and R³ are each independently hydrogen, C1 to C4 alkyl, hydroxy, aryl, or aryl substituted with one or more substituents selected from the group consisting of halo, nitro, cyano, C1 to C6 alkyl, C1 to C6 alkoxy, C1 to C6 halosubstituted alkyl, C1 to C6 hydroxysubstituted alkyl, C1 to C6 alkoxycarbonyl, aminocarbonyl, C1 to C6 alkylaminocarbonyl, di C1 to C6 alkylaminocarbonyl and C1 to C6 alkylsulfonyl, provided that R² and R³ are not both hydroxy;
R⁴ is hydrogen, a pharmaceutically acceptable cation, aroyl or C1 to C12 alkoyl;
A is C1 to C6 alkylene or C2 to C6 alkenylene, provided that A is not alpha to X when m is 0;
X is oxygen or sulfur;
each Y is independent hydrogen, halo, cyano, hydroxy, C1 to C12 alkyl, C1 to C12 alkoxy, C1 to C12 halosubstituted alkyl, C1 to C12 alklylthio, C2 to C12 alkenyl, C2 to C12 alkoxyalkyl, C3 to C8 cycloalkyl, aryl, aryloxy, C1 to C12 arylalkyl, C2 to C12 arylalkenyl, C1 to C12 arylalkoxy or any of the foregoing aryl-derived moieties substituted with one or more substituents selected from the group consisting of halo, nitro, cyano, C1 to C12 alkyl, C1 to C12 alkoxy, C1 to C12 alkylthio, C1 to C12 alkoyl, C1 to C12 halosubstituted alkyl, aryl, aroyl, aryloxy, arylthio, C1 to C12 arylalkyl, C1 to C12 alkylamino, di C1 to C12 alkylamino, C1 to C12 alkoxycarbonyl, aminocarbonyl, C1 to C12 alkylaminocarbonyl, di C1 to C12 alkylaminocarbonyl and any of the foregoing aroyl or aryl-derived substituents further substituted with one or more substituents selected from the group consisting of halo, nitro, cyano, C1 to C6 alkyl, C1 to C6 alkoxy, C1 to C6 alkylthio and C1 to C6 halosubstituted alkyl;
Z is oxygen or sulfur;
m is 0 or 1;
n is 1 to 5; and
p is 2 to 6, comprising:
(I) selectively hydrolyzing a compound having the formula 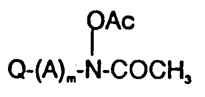 wherein Q is 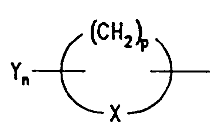 and X, Y, m, n and p are as defined above, with a base selected from ammonium hydroxide, sodium hydroxide, potassium hydroxide and lithium hydroxide in a solvent system under conditions including a reaction temperature of between -10°C and ambient temperature;
(II) reaction of a compound having the formula 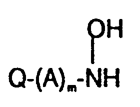 wherein Q is as defined above, with trimethylsilyl isocyanate in a reaction-inert solvent under conditions including a reaction temperature of between ambient and reflux temperature; or
(III) reaction of a compound having the formula 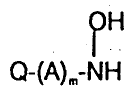 wherein Q is as defined above, with gaseous hydrogen chloride in a reaction-inert solvent under reaction conditions including a reaction temperature of between ambient temperature and boiling point of the solvent, followed by treatment with phosgene.

2. A process according to Claim 1 wherein:
when process (I) is used, said solvent system is selected from one or more of water, methanol, ethanol, propanol and tetrahydrofuran;
when process (II) is used, said reaction-inert solvent is selected from tetrahydrofuran, dioxane, methylene chloride and benzene; and
when process (III) is used, said reaction-inert solvent is selected from benzene and toluene.

3. A process according to Claim 1 or 2 further comprising the step of isolating said prepared compound.

4. A process according to one of Claims 1 to 3 wherein R⁴ is hydrogen.

5. A process according to one of Claims 1 to 4 wherein:
p is 4; and
X is oxygen.

6. A process according to one of Claims 1 to 5 wherein:
R¹ is NH₂; and
Z is oxygen.

7. A process according to one of Claims 1 to 6 wherein:
Y is aryl, substituted aryl or arylalkyl; and
n is 1.

## Patentansprüche (Patentansprüche für folgende(n) Vertragsstaat(en): AT, BE, CH, DE, DK, FR, GB, IT, LI, LU, NL, SE)

1. Verbindung mit der folgenden Formel: 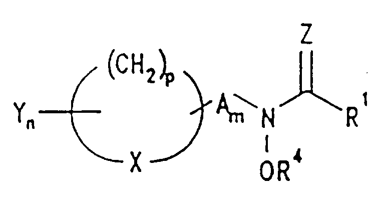 in der:
R¹ für Wasserstoff, (C₁-C₄)-Alkyl, (C₂-C₄)-Alkenyl oder NR²R³ steht;
R² und R³ jeweils unabhängig für Wasserstoff, (C₁-C₄)-Alkyl, Hydroxy, Aryl oder für Aryl stehen, das mit einem oder mehreren Substituenten substituiert ist, die aus folgendem ausgewählt sind: Halogen, Nitro, Cyano, (C₁-C₆)-Alkyl, (C₁-C₆)-Alkoxy, halogensubstituiertem (C₁-C₆)-Alkyl, hydroxysubstituiertem (C₁-C₆)-Alkyl, (C₁-C₆)-Alkoxycarbonyl, Aminocarbonyl, (C₁-C₆)-Alkylaminocarbonyl, Di-(C₁-C₆)-Alkylaminocarbonyl und (C₁-C₆)-Alkylsulfonyl, mit der Maßgabe, daß R² und R³ nicht beide für Hydroxy stehen;
R⁴ für Wasserstoff, ein pharmazeutisch geeignetes Kation, Aroyl oder (C₁-C₁₂)-Alkoyl steht;
A für (C₁-C₆)-Alkylen oder (C₂-C₆)-Alkenylen steht, mit der Maßgabe, daß A nicht alpha-ständig zu X ist, wenn m für 0 steht;
X für Sauerstoff oder Schwefel steht;
jedes Y unabhängig für folgendes steht: Wasserstoff, Halogen, Cyano, Hydroxy, (C₁-C₁₂)-Alkyl, (C₁-C₁₂)-Alkoxy, halogensubstituiertes (C₁-C₁₂)-Alkyl, (C₁-C₁₂)-Alkylthio, (C₂-C₁₂)-Alkenyl, (C₂-C₁₂)-Alkoxyalkyl, (C₃-C₈)-Cycloalkyl, Aryl, Aryloxy, (C₁-C₁₂)-Arylalkyl, (C₂-C₁₂)-Arylalkenyl, (C₁-C₁₂)-Arylalkoxy oder für jeden der obigen von Aryl abstammenden Reste, der mit einem oder mehreren Substituenten substituiert ist, die aus folgendem ausgewählt sind: Halogen, Nitro, Cyano, (C₁-C₁₂)-Alkyl, (C₁-C₁₂)-Alkoxy, (C₁-C₁₂)-Alkylthio, (C₁-C₁₂)-Alkoyl, halogensubstituiertem (C₁-C₁₂)-Alkyl, Aryl, Aroyl, Aryloxy, Arylthio, (C₁-C₁₂)-Arylalkyl, (C₁-C₁₂)-Alkylamino, Di-(C₁-C₁₂)-Alkylamino, (C₁-C₁₂)-Alkoxycarbonyl, Aminocarbonyl, (C₁-C₁₂)-Alkylaminocarbonyl, Di-(C₁-C₁₂)-Alkylaminocarbonyl und jedem der obigen von Aroyl oder Aryl abstammenden Substituenten, der außerdem mit einem oder mehreren Substituenten substituiert ist, die aus folgendem ausgewählt sind: Halogen, Nitro, Cyano, (C₁-C₆)-Alkyl, (C₁-C₆)-Alkoxy, (C₁-C₆)-Alkylthio und halogensubstituiertem (C₁-C₆)-Alkyl;
Z für Sauerstoff oder Schwefel steht;
m für 0 oder 1 steht;
n für 1 bis 5 steht; und
p für 2 bis 6 steht.

2. Verbindung nach Anspruch 1, wobei R⁴ für Wasserstoff steht.

3. Verbindung nach Anspruch 1 oder 2, wobei p für 4 steht und X für Sauerstoff steht.

4. Verbindung nach einem der Ansprüche 1 bis 3, wobei R¹ für NH₂ steht und Z für Sauerstoff steht.

5. Verbindung nach einem der Ansprüche 1 bis 4, wobei Y für Aryl, substituiertes Aryl oder Arylalkyl steht und n für 1 steht.

6. Verbindung nach Anspruch 5, die aus folgender Gruppe ausgewählt ist:
N-Hydroxy-N-(5-phenyltetrahydrofuran-2-yl)methylharnstoff;
N-Hydroxy-N-[5-(3-phenoxy)phenyltetrahydrofuran-2-yl]methylharnstoff;
N-Hydroxy-N-[5-(2-phenylethyl)tetrahydrofuran-2-yl]methylharnstoff;
N-Hydroxy-N-(4-phenyltetrahydrofuran-2-yl)methylharnstoff;
N-Hydroxy-N-[5-(4-phenoxyphenyl)tetrahydrofuran-2-yl]methylharnstoff;
und
N-Hydroxy-N-[4-(2-phenyl)tetrahydrofuranyl]harnstoff.

7. Verbindung nach Anspruch 6, die aus folgender Gruppe ausgewählt ist:
trans-N-Hydroxy-N-(5-phenyltetrahydrofuran-2-yl)methylharnstoff;
cis-N-Hydroxy-N-(5-phenyltetrahydrofuran-2-yl)methylharnstoff; und
trans-N-Hydroxy-N-[5-(3-phenoxy)phenyltetrahydrofuran-2-yl]methylharnstoff.

8. Verbindung nach Anspruch 5, die aus folgender Gruppe ausgewählt ist:
trans-N-Hydroxy-N-[5-(4-fluorphenyl)tetrahydrofuran-2-yl]methylharnstoff;
trans-N-Hydroxy-N-[5-(4-chlorphenyl)tetrahydrofuran-2-yl]methylharnstoff;
und
trans-N-Hydroxy-N-(5-[3-(4-methylphenoxy)phenyl]tetrahydrofuran-2-yl)methylharnstoff.

9. Pharmazeutische Zusammensetzung zur Behandlung allergischer oder entzündlicher Beschwerden eines Säugers, die eine therapeutisch wirksame Menge einer Verbindung nach einem der Ansprüche 1 bis 8, oder ein pharmazeutisch geeignetes Salz davon, und ein pharmazeutisch geeignetes Trägermittel enthält.

10. Pharmazeutische Zusammensetzung zur Behandlung von kardiovaskulären Erkrankungen eines Säugers, die eine therapeutisch wirksame Menge einer Verbindung nach einem der Ansprüche 1 bis 8, oder ein pharmazeutisch geeignetes Salz davon, und ein pharmazeutisch geeignetes Trägermittel enthält.

11. Verbindung nach einem der Ansprüche 1 bis 8, oder ein pharmazeutisch geeignetes Salz davon, oder eine pharmazeutische Zusammensetzung, die irgendeinen dieser Stoffe enthält, zur Verwendung als Arzneimittel.

12. Verwendung einer Verbindung nach einem der Ansprüche 1 bis 8, oder eines pharmazeutisch geeigneten Salzes davon, oder einer pharmazeutischen Zusammensetzung, die irgendeinen dieser Stoffe enthält, zur Herstellung eines Arzneimittels zur Hemmung von Lipoxygenase.

13. Verwendung einer Verbindung nach einem der Ansprüche 1 bis 8, oder eines pharmazeutisch geeigneten Salzes davon, oder einer pharmazeutischen Zusammensetzung, die irgendeinen dieser Stoffe enthält, zur Herstellung eines Arzneimittels zur Behandlung von Allergie oder entzündlichen Beschwerden.

14. Verwendung einer Verbindung nach einem der Ansprüche 1 bis 8, oder eines pharmazeutisch geeigneten Salzes davon, oder einer pharmazeutischen Zusammensetzung, die irgendeinen dieser Stoffe enthält, zur Herstellung eines Arzneimittels zur Behandlung von kardiovaskulären Erkrankungen.

## Patentansprüche (Patentansprüche für folgende(n) Vertragsstaat(en): ES, GR)

1. Verfahren zur Herstellung einer Verbindung mit der folgenden Formel: 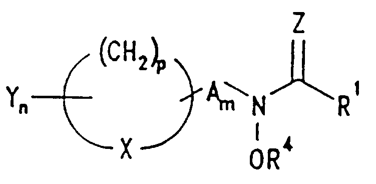 in der:
R¹ für Wasserstoff, (C₁-C₄)-Alkyl, (C₂-C₄)-Alkenyl oder NR²R³ steht;
R² und R³ jeweils unabhängig für Wasserstoff, (C₁-C₄)-Alkyl, Hydroxy, Aryl oder für Aryl stehen, das mit einem oder mehreren Substituenten substituiert ist, die aus folgendem ausgewählt sind: Halogen, Nitro, Cyano, (C₁-C₆)-Alkyl, (C₁-C₆)-Alkoxy, halogensubstituiertem (C₁-C₆)-Alkyl, hydroxysubstituiertem (C₁-C₆)-Alkyl, (C₁-C₆)-Alkoxycarbonyl, Aminocarbonyl, (C₁-C₆)-Alkylaminocarbonyl, Di-(C₁-C₆)-Alkylaminocarbonyl und (C₁-C₆)-Alkylsulfonyl, mit der Maßgabe, daß R² und R³ nicht beide für Hydroxy stehen;
R⁴ für Wasserstoff, ein pharmazeutisch geeignetes Kation, Aroyl oder (C₁-C₁₂)-Alkoyl steht;
A für (C₁-C₆)-Alkylen oder (C₂-C₆)-Alkenylen steht, mit der Maßgabe, daß A nicht alpha-ständig zu X ist, wenn m für 0 steht;
X für Sauerstoff oder Schwefel steht;
jedes Y unabhängig für folgendes steht: Wasserstoff, Halogen, Cyano, Hydroxy, (C₁-C₁₂)-Alkyl, (C₁-C₁₂)-Alkoxy, halogensubstituiertes (C₁-C₁₂)-Alkyl, (C₁-C₁₂)-Alkylthio, (C₂-C₁₂)-Alkenyl, (C₂-C₁₂)-Alkoxyalkyl, (C₃-C₈)-Cycloalkyl, Aryl, Aryloxy, (C₁-C₁₂)-Arylalkyl, (C₂-C₁₂)-Arylalkenyl, (C₁-C₁₂)-Arylalkoxy oder für jeden der obigen von Aryl abstammenden Reste, der mit einem oder mehreren Substituenten substituiert ist, die aus folgendem ausgewählt sind: Halogen, Nitro, Cyano, (C₁-C₁₂)-Alkyl, (C₁-C₁₂)-Alkoxy, (C₁-C₁₂)-Alkylthio, (C₁-C₁₂)-Alkoyl, halogensubstituiertem (C₁-C₁₂)-Alkyl, Aryl, Aroyl, Aryloxy, Arylthio, (C₁-C₁₂)-Arylalkyl, (C₁-C₁₂)-Alkylamino, Di-(C₁-C₁₂)-Alkylamino, (C₁-C₁₂)-Alkoxycarbonyl, Aminocarbonyl, (C₁-C₁₂)-Alkylaminocarbonyl, Di-(C₁-C₁₂)-Alkylaminocarbonyl und jedem der obigen von Aroyl oder Aryl abstammenden Substituenten, der außerdem mit einem oder mehreren Substituenten substituiert ist, die aus folgendem ausgewählt sind: Halogen, Nitro, Cyano, (C₁-C₆)-Alkyl, (C₁-C₆)-Alkoxy, (C₁-C₆)-Alkylthio und halogensubstituiertem (C₁-C₆)-Alkyl;
Z für Sauerstoff oder Schwefel steht;
m für 0 oder 1 steht;
n für 1 bis 5 steht; und
p für 2 bis 6 steht, bei dem:
(I) eine Verbindung mit der folgenden Formel 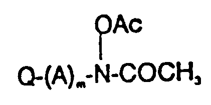 in der Q für 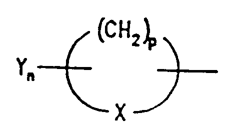 steht und X, Y, m, n und p wie oben definiert sind, mit einer Base selektiv hydrolysiert wird, die aus Ammoniumhydroxid, Natriumhydroxid, Kaliumhydroxid und Lithiumhydroxid ausgewählt ist, und zwar in einem Lösungsmittelsystem unter Bedingungen, die eine Reaktionstemperatur von zwischen -10°C und Raumtemperatur einschließen;
(II) eine Verbindung mit der folgenden Formel 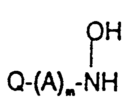 in der Q wie oben definiert ist, mit Trimethylsilylisocyanat in einem reaktionsinerten Lösungsmittel unter Bedingungen umgesetzt wird, die eine Reaktionstemperatur zwischen Raum- und Rückflußtemperatur einschließen; oder
(III) eine Verbindung mit der folgenden Formel 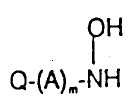 in der Q wie oben definiert ist, mit gasförmigem Chlorwasserstoff in einem reaktionsinerten Lösungsmittel unter Reaktionsbedingungen umgesetzt wird, die eine Reaktionstemperatur zwischen Raumtemperatur und dem Siedepunkt des Lösungsmittels einschließen, gefolgt von der Behandlung mit Phosgen.

2. Verfahren nach Anspruch 1, bei dem:
wenn Verfahren (I) verwendet wird, das Lösungsmittelsystem aus einem oder mehreren der folgenden ausgewählt ist: Wasser, Methanol, Ethanol, Propanol und Tetrahydrofuran;
wenn Verfahren (II) verwendet wird, das reaktionsinerte Lösungsmittel aus Tetrahydrofuran, Dioxan, Methylenchlorid und Benzol ausgewählt ist; und
wenn Verfahren (III) verwendet wird, das reaktionsinerte Lösungsmittel aus Benzol und Toluol ausgewählt ist.

3. Verfahren nach Anspruch 1 oder 2, das außerdem den Schritt aufweist, daß die hergestellte Verbindung isoliert wird.

4. Verfahren nach einem der Ansprüche 1 bis 3, wobei R⁴ für Wasserstoff steht.

5. Verfahren nach einem der Ansprüche 1 bis 4, wobei p für 4 steht und X für Sauerstoff steht.

6. Verfahren nach einem der Ansprüche 1 bis 5, wobei R¹ für NH₂ steht und Z für Sauerstoff steht.

7. Verfahren nach einem der Ansprüche 1 bis 6, wobei Y für Aryl, substituiertes Aryl oder Arylalkyl steht und n für 1 steht.

## Revendications (Revendications pour l'(les) Etat(s) contractant(s) suivant(s): AT, BE, CH, DE, DK, FR, GB, IT, LI, LU, NL, SE)

1. Composé de formule 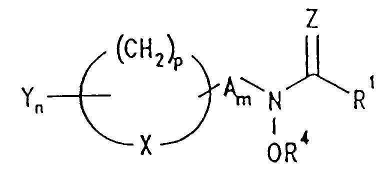 dans laquelle :
R¹ représente l'hydrogène, un groupe alkyle en C₁ à C₄, alcényle en C₂ à C₄ ou NR²R³ ;
R² et R³ représentent chacun indépendamment l'hydrogène, un groupe alkyle en C₁ à C₄, hydroxy, aryle, ou aryle portant un ou plusieurs substituants choisis entre des groupes halogéno, nitro, cyano, alkyle en C₁ à C₆, alkoxy en C₁ à C₆, alkyle en C₁ à C₆ substitué avec un halogène, alkyle en C₁ à C₆ substitué avec un groupe hydroxy, (alkoxy en C₁ à C₆)-carbonyle, aminocarbonyle, (alkyle en C₁ à C₆)-aminocarbonyle, di(alkyle en C₁ à C₆)-aminocarbonyle et alkylsulfonyle en C₁ à C₆, sous réserve que R² et R³ ne représentent pas l'un et l'autre un groupe hydroxy ;
R⁴ représente l'hydrogène, un cation pharmaceutiquement acceptable, un groupe aroyle ou alkoyle en C₁ à C₁₂ ;
A représente un groupe alkylène en C₁ à C₆ ou alcénylène en C₂ à C₆, sous réserve que A ne soit pas en position alpha par rapport à X lorsque m est égal à zéro ;
X représente l'oxygène ou le soufre,
chaque groupe Y représente indépendamment l'hydrogène, un groupe halogéno, cyano, hydroxy, alkyle en C₁ à C₁₂, alkoxy en C₁ à C₁₂, alkyle en C₁ à C₁₂ substitué avec un halogène, alkylthio en C₁ à C₁₂, alcényle en C₂ à C₁₂, alkoxyalkyle en C₂ à C₁₂, cycloalkyle en C₃ à C₈, aryle, aryloxy, arylalkyle en C₁ à C₁₂, arylalcényle en C₂ à C₁₂, arylalkoxy en C₁ à C₁₂ ou n'importe lequel des groupements précités dérivés d'un groupe aryle, portant un ou plusieurs substituants choisis entre des groupes halogéno, nitro, cyano, alkyle en C₁ à C₁₂, alkoxy en C₁ à C₁₂, alkylthio en C₁ à C₁₂, alkoyle en C₁ à C₁₂, alkyle en C₁ à C₁₂ substitué avec un halogène, aryle, aroyle, aryloxy, arylthio, arylalkyle en C₁ à C₁₂, alkylamino en C₁ à C₁₂, di(alkyle en C₁ à C₁₂)-amino, (alkoxy en C₁ à C₁₂)-carbonyle, aminocarbonyle, (alkyle en C₁ à C₁₂)-aminocarbonyle, di(alkyle en C₁ à C₁₂)-aminocarbonyle et n'importe lequel des substituants précités dérivés d'un groupe aroyle ou aryle, portant en outre un ou plusieurs substituants choisis entre des groupes halogéno, nitro, cyano, alkyle en C₁ à C₆, alkoxy en C₁ à C₆, alkylthio en C₁ à C₆ et alkyle en C₁ à C₆ substitué avec un halogène ;
Z représente l'oxygène ou le soufre ;
m est égal à zéro ou 1 ;
n a une valeur de 1 à 5 ; et
p a une valeur de 2 à 6.

2. Composé suivant la revendication 1, dans lequel R⁴ représente l'hydrogène.

3. Composé suivant la revendication 1 ou 2, dans lequel :
p est égal à 4 ; et
X représente l'oxygène.

4. Composé suivant l'une quelconque des revendications 1 à 3, dans lequel :
R¹ représente un groupe NH₂ ; et
Z représente l'oxygène.

5. Composé suivant l'une quelconque des revendications 1 à 4, dans lequel :
Y représente un groupe aryle, aryle substitué ou arylalkyle ; et
n est égal à 1.

6. Composé suivant la revendication 5, choisi dans le groupe consistant en :
la N-hydroxy-N-(5-phényltétrahydrofuran-2-yl)méthylurée ;
la N-hydroxy-N-[5-(3-phénoxy)phényltétrahydrofuran-2-yl]méthylurée ;
la N-hydroxy-N-[5-(2-phényléthyl)tétrahydrofuran-2-yl]méthylurée ;
la N-hydroxy-N-(4-phényltétrahydrofuran-2-yl)méthylurée ;
la N-hydroxy-N-[5-(4-phénoxyphényl)tétrahydrofuran-2-yl]méthylurée ; et
la N-hydroxy-N-[4-(2-phényl)tétrahydrofuranyl]urée.

7. Composé suivant la revendication 6, choisi dans le groupe consistant en :
la trans-N-hydroxy-N-(5-phényltétrahydrofuran-2-yl)méthylurée ;
la cis-N-hydroxy-N-(5-phényltétrahydrofuran-2-yl)méthylurée ; et
la trans-N-hydroxy-N-[5-(3-phénoxy)phényltétrahydrofuran-2-yl]méthylurée.

8. Composé suivant la revendication 5, choisi dans le groupe consistant en :
la trans-N-hydroxy-N-[5-(4-fluorophényl)tétrahydrofuran-2-yl)]méthylurée ;
la trans-N-hydroxy-N-[5-(4-chlorophényl)tétrahydrofuran-2-yl)méthylurée ; et
la trans-N-hydroxy-N-(5-[3-(4-méthylphénoxy)-phényl]tétrahydrofuran-2-yl]méthylurée.

9. Composition pharmaceutique pour le traitement d'affections allergiques ou inflammatoires chez un mammifère, comprenant une quantité à effet thérapeutique d'un composé suivant une des revendications 1 à 8, ou d'un de ses sels pharmaceutiquement acceptables, et un support pharmaceutiquement acceptable.

10. Composition pharmaceutique pour le traitement de maladies cardiovasculaires chez un mammifère, comprenant une quantité à effet thérapeutique d'un composé suivant une des revendications 1 à 8, ou d'un de ses sels pharmaceutiquement acceptables, et un support pharmaceutiquement acceptable.

11. Composé suivant l'une quelconque des revendications 1 à 8, ou un de ses sels pharmaceutiquement acceptables, ou bien une composition pharmaceutique contenant l'une ou l'autre entité, destinée à être utilisée comme médicament.

12. Utilisation d'un composé suivant l'une quelconque des revendications 1 à 8, ou d'un de ses sels pharmaceutiquement acceptables, ou bien d'une composition pharmaceutique contenant l'une ou l'autre entité, pour la production d'un médicament destiné à inhiber la lipoxygénase.

13. Utilisation d'un composé suivant l'une quelconque des revendications 1 à 8, ou d'un de ses sels pharmaceutiquement acceptables, ou bien d'une composition pharmaceutique contenant l'une ou l'autre entité, pour la production d'un médicament destiné au traitement de l'allergie ou d'affections inflammatoires.

14. Utilisation d'un composé suivant l'une quelconque des revendications 1 à 8, ou d'un de ses sels pharmaceutiquement acceptables, ou bien d'une composition pharmaceutique contenant l'une ou l'autre entité, pour la production d'un médicament destiné au traitement de maladies cardiovasculaires.

## Revendications (Revendications pour l'(les) Etat(s) contractant(s) suivant(s): ES, GR)

1. Procédé de préparation d'un composé de formule 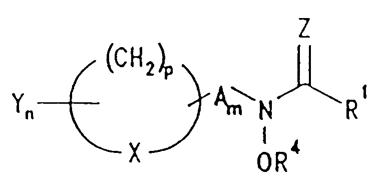 dans laquelle :
R¹ représente l'hydrogène, un groupe alkyle en C₁ à C₄, alcényle en C₂ à C₄ ou NR²R³ ;
R² et R³ représentent chacun indépendamment l'hydrogène, un groupe alkyle en C₁ à C₄, hydroxy, aryle, ou aryle portant un ou plusieurs substituants choisis entre des groupes halogéno, nitro, cyano, alkyle en C₁ à C₆, alkoxy en C₁ à C₆, alkyle en C₁ à C₆ substitué avec un halogène, alkyle en C₁ à C₆ substitué avec un groupe hydroxy, (alkoxy en C₁ à C₆)-carbonyle, aminocarbonyle, (alkyle en C₁ à C₆)-aminocarbonyle, di(alkyle en C₁ à C₆)-aminocarbonyle et alkylsulfonyle en C₁ à C₆, sous réserve que R² et R³ ne représentent pas l'un et l'autre un groupe hydroxy ;
R⁴ représente l'hydrogène, un cation pharmaceutiquement acceptable, un groupe aroyle ou alkoyle en C₁ à C₁₂ ;
A représente un groupe alkylène en C₁ à C₆ ou alcénylène en C₂ à C₆, sous réserve que A ne soit pas en position alpha par rapport à X lorsque m est égal à zéro ;
X représente l'oxygène ou le soufre,
chaque groupe Y représente indépendamment l'hydrogène, un groupe halogéno, cyano, hydroxy, alkyle en C₁ à C₁₂, alkoxy en C₁ à C₁₂, alkyle en C₁ à C₁₂ substitué avec un halogène, alkylthio en C₁ à C₁₂, alcényle en C₂ à C₁₂, alkoxyalkyle en C₂ à C₁₂, cycloalkyle en C₃ à C₈, aryle, aryloxy, arylalkyle en C₁ à C₁₂, arylalcényle en C₂ à C₁₂, arylalkoxy en C₁ à C₁₂ ou n'importe lequel des groupements précités dérivés d'un groupe aryle, portant un ou plusieurs substituants choisis entre des groupes halogéno, nitro, cyano, alkyle en C₁ à C₁₂, alkoxy en C₁ à C₁₂, alkylthio en C₁ à C₁₂, alkoyle en C₁ à C₁₂, alkyle en C₁ à C₁₂ substitué avec un halogène, aryle, aroyle, aryloxy, arylthio, arylalkyle en C₁ à C₁₂, alkylamino en C₁ à C₁₂, di(alkyle en C₁ à C₁₂)-amino, (alkoxy en C₁ à C₁₂)-carbonyle, aminocarbonyle, (alkyle en C₁ à C₁₂)-aminocarbonyle, di(alkyle en C₁ à C₁₂)-aminocarbonyle et n'importe lequel des substituants précités dérivés d'un groupe aroyle ou aryle, portant en outre un ou plusieurs substituants choisis entre des groupes halogéno, nitro, cyano, alkyle en C₁ à C₆, alkoxy en C₁ à C₆, alkylthio en C₁ à C₆ et alkyle en C₁ à C₆ substitué avec un halogène ;
Z représente l'oxygène ou le soufre ;
m est égal à zéro ou 1 ;
n a une valeur de 1 à 5 ; et
p a une valeur de 2 à 6, comprenant :
(I) l'hydrolyse sélective d'un composé répondant à la formule : 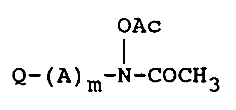 dans laquelle Q représente un groupe 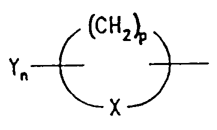 et X, Y, m, n et p répondent aux définitions précitées, avec une base choisie entre l'hydroxyde d'ammonium, l'hydroxyde de sodium, l'hydroxyde de potassium et l'hydroxyde de lithium dans un solvant dans des conditions comprenant une température de réaction comprise dans l'intervalle de -10°C à la température ambiante ;
(II) la réaction d'un composé répondant à la formule 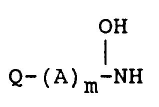 dans laquelle Q répond à la définition précitée, avec l'isocyanate de triméthylsilyle dans un solvant inerte vis-à-vis de la réaction, dans des conditions comprenant une température de réaction comprise dans l'intervalle de la température ambiante à la température du reflux ; ou
(III) la réaction d'un composé répondant à la formule 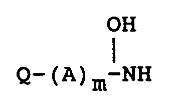 dans laquelle Q répond à la définition précitée, avec le chlorure d'hydrogène gazeux dans un solvant inerte vis-à-vis de la réaction, dans des conditions de réaction comprenant une température de réaction comprise dans l'intervalle de la température ambiante au point d'ébullition du solvant, puis un traitement avec du phosgène.

2. Procédé suivant la revendication 1, dans lequel :
lorsque le procédé (I) est utilisé, le solvant est choisi entre un ou plusieurs des composés consistant en l'eau, le méthanol, l'éthanol, le propanol et le tétrahydrofuranne ;
lorsque le procédé (II) est utilisé, le solvant inerte vis-à-vis de la réaction est choisi entre le tétrahydrofuranne, le dioxane, le chlorure de méthylène et le benzène ; et
lorsque le procédé (III) est utilisé, le solvant inerte vis-à-vis de la réaction est choisi entre le benzène et le toluène.

3. Procédé suivant la revendication 1 ou 2, comprenant en outre l'étape d'isolement du composé préparé.

4. Procédé suivant une des revendications 1 à 3, dans lequel R⁴ représente l'hydrogène.

5. Procédé suivant une des revendications 1 à 4, dans lequel :
p est égal à 4 ; et
X représente l'oxygène.

6. Procédé suivant une des revendications 1 à 5, dans lequel :
R¹ représente un groupe NH₂ ; et
Z représente l'oxygène.

7. Procédé suivant une des revendications 1 à 6, dans lequel :
Y représente un groupe aryle, aryle substitué ou arylalkyle ; et
n est égal à 1.
